# EUROPEAN PATENT SPECIFICATION

(11) **EP 1 532 124 B1**
(45) Date of publication and mention of the grant of the patent: **19.07.2006**
(21) Application number: 03761003.7
(22) Date of filing: 23.06.2003
(51) Int. Cl.: C07D 295/182, C07D 211/66, C07D 217/12, C07D 309/32, A61K 31/495, A61K 31/5375, A61K 31/472, A61P 37/00

(54) **Use of nitrile derivatives as medicament.**
Verwendung von Nitrilenverbindungen als Arztneimittel
Utilisation de nitriles comme médicament.

(30) Priority: 24.06.2002 SE 0201977
(43) Date of publication of application: 25.05.2005
(73) Proprietor: AstraZeneca AB, 151 85 Södertälje (SE)
(72) Inventor: BAILEY, Andrew, AstraZeneca R & D Charnwood, Loughborough, Leicestershire LE11 5RH (GB); PAIRAUDEAU, Garry, AstraZeneca R & D Charnwood, Loughborough, Leicestershire LE11 5RH (GB); PATEL, Anil, AstraZeneca R & D Charnwood, Loughborough, Leicestershire LE11 5RH (GB); THOM, Stephen, AstraZeneca R & D Charnwood, Loughborough, Leicestershire LE11 5RH (GB)
(86) International application number: PCT/SE2003/001080
(87) International publication number: WO 2004/000825

(56) References cited:
- WO-A1-01/09110
- US-A1- 2001 041 700

## Description

The present invention relates to compounds and compositions for treating diseases associated with cysteine protease activity. The compounds are reversible inhibitors of cysteine proteases S, K, F, L and B. Of particular interest are diseases associated with Cathepsin S. In addition this invention also discloses processes for the preparation of such inhibitors.

### BACKGROUND OF THE INVENTION

Cathepsin S is a member of the papain superfamily of cysteine proteases which also encompasses Cathepsins B, H, L, O and K. Cathepsin S plays a key role in the processing of invariant chain in MHC class II complexes allowing the complex to associate with antigenic peptides. MHC class II complexes are then transported to the surface of the cell for presentation to effector cells such as T cells. The process of antigen presentation is a fundamental step in initiation of the immune response. In this respect inhibitors of cathepsin S could be useful agents in the treatment of inflammation and immune disorders such as, but not limited to, asthma, rheumatoid arthritis, multiple sclerosis and Crohn's disease. Cathepsin S has also been implicated in a variety of other diseases involving extracellular proteolysis such as the development of emphysema in COPD through degradation of elastin and in Alzheimers disease.

Other Cathepsins notably K and L have been shown to degrade bone collagen and other bone matrix proteins. Inhibitors of these cysteine proteases would be expected to be useful in the treatment of diseases involving bone resorption such as osteoporosis.

The present invention therefore provides use of a compound of formula (I) 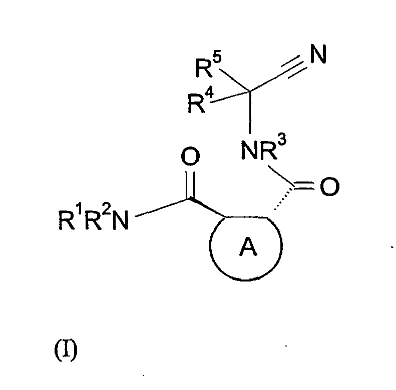 in which:
A is a 6-membered ring optionally containing a double bond and optionally containing an oxygen atom or NR group in the ring;
R is hydrogen or C₁₋₆ alkyl;
R¹ and R² are independently, C₁₋₆ alkyl or C₃₋₆ cycloalkyl both of which can optionally contain one or more O, S or NR³ groups, or R¹ and R² together with the nitrogen atom to which they are attached form a 3,4-dihydroisoquinoline ring or a 5- or 6-membered saturated ring optionally containing a further O, S or N atom and optionally substituted by a group -(CH₂)ₚ-R⁶ where p is 0 to 3 and R⁶ is C₁₋₆ alkyl, CONR⁷R⁸ where R⁷ and R⁸ are independently hydrogen, C₁₋₆ alkyl which can optionally contain one or more O, S or NR³ groups, or together with the nitrogen atom to which they are attached form a 5- or 6-membered saturated ring optionally containing a further O, S or NR³ group;
or R⁶ is a 4 to 7-membered saturated ring optionally containing one or more O, S or N atoms, or an aryl or heteroaryl group containing one to four heteroatoms selected from O, S or N, the saturated ring, aryl and heteroaryl groups all being optionally substituted by halogen, amino, hydroxy, cyano, nitro, carboxy, CONR⁷R⁸, SO₂NR⁷R⁸, SO₂R³, trifluoromethyl, NHSO₂R³, NHCOR³, C₁₋₆ alkyl, C₁₋₆ alkoxy, SR³ or NR⁹R¹⁰ where R⁹ and R¹⁰ are independently hydrogen, C₁₋₆ alkyl or together with the nitrogen atom to which they are attached form a 5- or 6-membered saturated ring optionally containing a further O, S or NR³ group;
R³ is hydrogen or C₁₋₆ alkyl;
R⁴ is hydrogen or C₁₋₆ alkyl;
R⁵ is hydrogen, C₁₋₆ alkyl or C₃₋₆ cycloalkyl both of which can optionally contain one or more O, S or NR³ groups or R⁵ is aryl or a 5- or 6-membered heteroaryl group containing one or two heteroatoms selected from O, S or N, the aryl and heteroaryl groups all being optionally substituted by halogen, amino, hydroxy, cyano, nitro, carboxy, CONR⁷R⁸, SO₂NR⁷R⁸, SO₂R³, trifluoromethyl, NHSO₂R³, NHCOR³, C₁₋₆ alkyl, C₁₋₆ alkoxy, SR³ or NR⁹R¹⁰ where R⁹ and R¹⁰ are independently hydrogen, C₁₋₆ alkyl or together with the nitrogen atom to which they are attached form a 5- or 6-membered saturated ring optionally containing a further O, S or NR³ group;
or R⁴ and R⁵ together form a 5- or 6-membered saturated ring optionally containing a further O, S or NR³ group and optionally substituted by, C₁₋₆ alkyl;
and pharmaceutically acceptable salts or solvates thereof, in the manufacture of a medicament for use in the treatment of inflammation and immune disorders in a warm blooded animal, such as man.

In the context of the present specification, unless otherwise indicated, an alkyl or alkenyl group or an alkyl or alkenyl moiety in a substituent group may be linear or branched. Aryl groups include phenyl and naphthyl. Heteroaryl groups include 5- or 6-membered, 5,6- or 6,6-fused aromatic rings containing one or more heteroatoms selected from N, S, O. Examples include pyridine, pyrimidine, pyrazine, pyridazine thiazole, oxazole, pyrazole, imidazole, furan and thiophene, quinoline, isoquinoline, benzimidazole, benzofuran, benzothiophene, indole.

Certain compounds of formula (I) are capable of existing in stereoisomeric forms. It will be understood that the invention encompasses all geometric and optical isomers of the compounds of formula (I) and mixtures thereof including racemates. Tautomers and mixtures thereof also form an aspect of the present invention.

Suitably A is a 6-membered ring optionally containing a double bond and optionally containing an oxygen atom or NR group in the ring where R is hydrogen or C₁₋₆ alkyl. A double bond can be present in any suitable position of the ring A. An oxygen atom or NR group can be present in any suitable position of the ring A, in addition to a double bond if desired. Preferably A is a cyclohexane ring.

Preferably R¹ and R² together with the nitrogen atom to which they are attached form an unsubstituted morpholine ring or a piperidine or piperazine ring substituted by a group -(CH₂)ₚ-R⁶ where p and R⁶ are as defined above. Preferably p is 0 and R⁶ is aryl or heteroaryl optionally substituted as defined above.

Preferably R³ is hydrogen.

Preferably R⁴ is hydrogen.

Preferably R⁵ is hydrogen, phenyl optionally substituted by C₁₋₆ alkyl or C₁₋₆ alkoxy

Preferred compounds of the invention include:
(1R,2R)-N-[Cyano(2-methoxyphenyl)methyl]-2-(morpholin-4-ylcarbonyl)cyclohexanecarboxamide,
(1R,2R)-N-[Cyano(2-methoxyphenyl)methyl]-2-{[4-(4-fluorobenzyl)piperazin-1-yl]carbonyl}cyclohexane carboxamide,
(1R,2R)-N-[Cyano(2-methoxyphenyl)methyl]-2-(3,4-dihydroisoquinolin-2(1H)-ylcarbonyl)cyclohexane carboxamide,
(±) Trans-N-(cyanomethyl)-2-{[4-(4-fluorobenzyl)piperazin-1-yl]carbonyl}cyclohexanecarboxamide,
(±) Trans-N-[cyano(2-methoxyphenyl)methyl]-2-[(4-methylpiperazin-1-yl)carbonyl]cyclohexanecarboxamide,
(1R,2R)-N-[Cyano(2-methoxyphenyl)methyl]-2-{[4-(4-fluorophenyl)piperazin-1-yl]carbonyl}cyclohexane carboxamide,
(1R,2R)-N-(4-Cyano-1-methylpiperidin-4-yl)-2-{[4-(4-fluorophenyl)piperazin-1-yl]carbonyl}cyclohexane carboxamide,
(1R,2R)-N-(4-Cyanotetrahydro-2H-pyran-4-yl)-2-{[4-(4-fluorophenyl)piperazin-1-yl]carbonyl}cyclohexane carboxamide,
(1R,2R)-N-[(1S)-1-cyano-3-methoxypropyl]-2-{[4-(4-fluorophenyl)piperazin-1-yl]carbonyl}cyclohexanecarboxamide,
and pharmaceutically acceptable salts thereof.

The present invention further provides a process for the preparation of a compound of formula (I) which comprises reaction of a compound of general formula (II) with a dehydrating agent (e.g. phosphorous oxychloride)

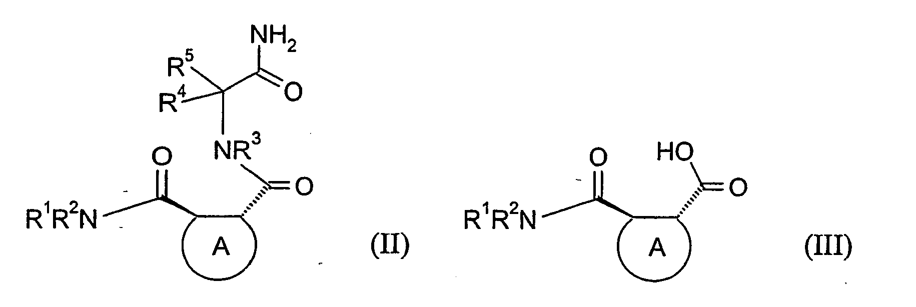

Compounds of formula (II) may be prepared from compounds of formula (III) by activation of the acid with an appropriate coupling agent or formation of acid chloride followed by reaction with an amine HNR³(CR⁴R⁵)CONH₂ where R³, R⁴ and R⁵ are defined in formula (I)
reaction of a compound of general formula (III) by activation of the acid group with an appropriate coupling agent or formation of acid chloride followed by reaction with an amine HNR³(CR⁴R⁵)CN where R³, R⁴ and R⁵ are defined in formula (I)
reaction of a compound of general formula (IV), where X= CN or CONH₂, by activation of the acid group with an appropriate coupling agent or formation of acid chloride followed by reaction with an amine HNR¹R² where R¹ and R² are defined in formula (I)

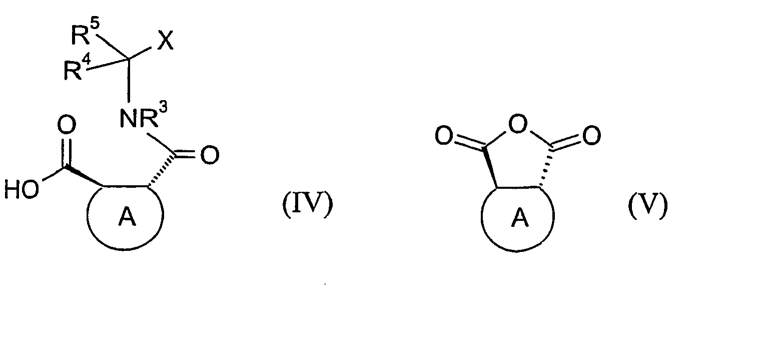

Compounds of general formula (III) and (IV) may be prepared from compound of general formula (V) by reaction with an amine of general formula HNR³(CR⁴R⁵)CN, HNR³(CR⁴R⁵)CONH₂ where R³, R⁴ and R⁵ are defined in formula (I) or HNR¹R² where R¹ and R² are defined in formula (I).

Compounds of general formula (III) and (IV) may also be prepared from a compound of general formula (VI) by activation of the acid group with an appropriate coupling agent or formation of acid chloride followed by reaction with an amine of general formula HNR³(CR⁴R⁵)CN, HNR³(CR⁴R⁵)CONH₂ where R³, R⁴ and R⁵ are defined in formula (I) or HNR¹R² where R¹ and R² are defined in formula (I) followed by hydrolysis of the ester.

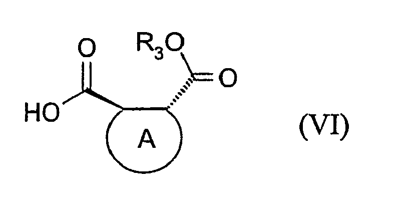

According to a further feature of the invention there is provided a compound of the formula **(I)**, or a pharmaceutically acceptable salt thereof, for use as a therapeutic agent.

According to a further feature of the present invention there is provided a method for producing inhibition of a cysteine protease in a warm blooded animal, such as man, in need of such treatment, which comprises administering to said animal an effective amount of a compound of the present invention, or a pharmaceutically acceptable salt thereof. In particular the compounds of the invention are useful in the treatment of inflammation and immune disorders such as, but not limited to, asthma, rheumatoid arthritis, COPD, multiple sclerosis, Crohn's disease, Alzheimers and pain, such as neuropathic pain. Preferably the compounds of the invention are used to treat pain, especially neuropathic pain.

The invention also provides a compound of the formula (I), or a pharmaceutically acceptable salt thereof, for use as a medicament; and the use of a compound of the formula **(I)** of the present invention, or a pharmaceutically acceptable salt thereof, in the manufacture of a medicament for use in the inhibition of a cysteine protease in a warm blooded animal, such as man.

In particular the invention provides the use of a compound of the formula **(I)** of the present invention, or a pharmaceutically acceptable salt thereof, in the manufacture of a medicament for use in the inhibition of Cathepsin S in a warm blooded animal, such as man. In order to use a compound of the formula **(I)** or a pharmaceutically acceptable salt thereof for the therapeutic treatment of mammals including humans, in particular in the inhibition of a cysteine protease, it is normally formulated in accordance with standard pharmaceutical practice as a pharmaceutical composition.

Therefore in another aspect the present invention provides a pharmaceutical composition which comprises a compound of the formula **(I)** or a pharmaceutically acceptable salt thereof and a pharmaceutically acceptable diluent or carrier.

The pharmaceutical compositions of this invention may be administered in standard manner for the disease condition that it is desired to treat, for example by oral, rectal or parenteral administration. For these purposes the compounds of this invention may be formulated by means known in the art into the form of, for example, tablets, capsules, aqueous or oily solutions or suspensions, (lipid) emulsions, dispersible powders, suppositories, ointments, creams, drops and sterile injectable aqueous or oily solutions or suspensions.

A suitable pharmaceutical composition of this invention is one suitable for oral administration in unit dosage form, for example a tablet or capsule which contains between 100 mg and 1 g of the compound of this invention.

In another aspect a pharmaceutical composition of the invention is one suitable for intravenous, subcutaneous or intramuscular injection.

Each patient may receive, for example, an intravenous, subcutaneous or intramuscular dose of 1 mgkg⁻¹ to 100 mgkg⁻¹ of the compound, preferably in the range of 5 mgkg⁻¹ to 20 mgkg⁻¹ of this invention, the composition being administered 1 to 4 times per day. The intravenous, subcutaneous and intramuscular dose may be given by means of a bolus injection. Alternatively the intravenous dose may be given by continuous infusion over a period of time. Alternatively each patient will receive a daily oral dose which is approximately equivalent to the daily parenteral dose, the composition being administered 1 to 4 times per day.

The following illustrate representative pharmaceutical dosage forms containing the compound of formula **(I)**, or a pharmaceutically-acceptable salt thereof (hereafter compound X), for therapeutic or prophylactic use in humans:
(a)

| Tablet I | mg/tablet |
|---|---|
| Compound X. | 100 |
| Lactose Ph.Eur. | 179 |
| Croscarmellose sodium | 12.0 |
| Polyvinylpyrrolidone | 6 |
| Magnesium stearate | 3.0 |

(b)

| Tablet II | mg/tablet |
|---|---|
| Compound X | 50 |
| Lactose Ph.Eur. | 229 |
| Croscarmellose sodium | 12.0 |
| Polyvinylpyrrolidone | 6 |
| Magnesium stearate | 3.0 |

(c)

| Tablet III | mg/tablet |
|---|---|
| Compound X | 1.0 |
| Lactose Ph.Eur. | 92 |
| Croscarmellose sodium | 4.0 |
| Polyvinylpyrrolidone | 2.0 |
| Magnesium stearate | 1.0 |

(d)

| Capsule | mg/capsule |
|---|---|
| Compound X | 10 |
| Lactose Ph.Eur. | 389 |
| Croscarmellose sodium | 100 |
| Magnesium stearate | 1. |

(e)

| Injection I | (50 mg/ml) |
|---|---|
| Compound X | 5.0% w/v |
| Isotonic aqueous solution | to 100% |

Buffers, pharmaceutically-acceptable cosolvents such as polyethylene glycol, polypropylene glycol, glycerol or ethanol or complexing agents such as hydroxy-propyl β cyclodextrin may be used to aid formulation.

### Note

The above formulations may be obtained by conventional procedures well known in the pharmaceutical art. The tablets (a)-(c) may be enteric coated by conventional means, for example to provide a coating of cellulose acetate phthalate.

The following examples illustrate the invention.

### Example 1

### (1R,2R)-N-[Cyano(2-methoxyphenyl)methyl]-2-(morpholin-4-ylcarbonyl)cyclohexanecarboxamide

### (i) (1R,2R)-2-({[Cyano(2-methoxyphenyl)methyl]amino}carbonyl) cyclohexanecarboxylic acid

A mixture of (3aR,7aR)-hexahydro-2-benzofuran-1,3-dione (3.64g), N,N-diisopropylethylamine (7.63g) and (±) 2-(2-methoxyphenyl)aminoacetonitrile hydrochloride (4.7g) in tetrahydrofuran (50ml) was stirred at room temperature for 6 hours. The solvent was removed under reduced pressure and the residue dissolved in water. The cooled (0°C) aqueous solution was acidified by dropwise addition of dilute aqueous hydrochloric acid and the resultant mixture extracted with ethyl acetate. The organic layer was washed with aqueous brine, dried (MgSO4), and evaporated under reduced pressure. The residue was purified by tritration with diethyl ether (100ml) followed by ethyl acetate (3 x 30ml). Yield 1.5g
MS: APCI(+ve) 317(M+1)

### (ii) (1R,2R)-N-[Cyano(2-methoxyphenyl)methyl]-2-(morpholin-4-ylcarbonyl)cyclohexanecarboxamide

A solution of the product from step (i) (0.35g), morpholine (0.14g), N,N-diisopropylethylamine (0.36g) and 1-hydroxybenzotriazole (0.22g) in tetrahydrofuran (10ml) was treated with N-(3-dimethylaminopropyl)-N'-ethylcarbodiimide hydrochloride (0.32g) and stirred for 6 hours at room temperature. The mixture was partitioned between ethyl acetate and aqueous brine, the organics dried (MgSO4) and evaporated under reduced pressure. The residue was purified by chromatography on silica, eluting with a mixture of ethyl acetate (75%) and isohexane (25%). Yield 0.05g
MS: APCI(+ve) 386(M+1)
1H NMR: (CDCl₃) δ 7.37-7.29(2H, m), 6.96-6.92(3H, m), 6.07(1H, d), 3.97(3H, s), 3.53-3.28(6H, m), 3.16-3.12(2H, m), 2.77-1.63(2H, m), 1.95(1H, d), 1.84-1.58(4H, m), 1.46-1.26(3H, m).

### Examples 2 and 3

Examples 2 and 3 were prepared according to the general method of example 1 using the appropriate amines

### Example 2

### (1R,2R)-N-[Cyano(2-methoxyphenyl)methyl]-2-{[4-(4-fluorobenzyl)piperazin-1-yl]carbonyl}cyclohexane carboxamide.

MS: APCI(+ve) 493(M+1)
1H NMR: (CDCl₃) δ 7.37-7.21(4H, m), 7.03-6.92(5H, m), 6.06(1H, d), 3.96(3H, s), 3.36-3.31(5H, m), 3.20-3.10(1H, m), 2.73-2.66(2H, m), 2.29-2.25(2H, m), 2.20-2.12(1H, m), 1.95-1.60(6H, m), 1.40-1.20(3H, m).

### Example 3

### (1R,2R)-N-[Cyano(2-methoxyphenyl)methyl]-2-(3,4-dihydroisoquinolin-2(1H)-ylcarbonyl)cyclohexane carboxamide

MS: APCI(+ve) 432(M+1)
1H NMR: (DMSO-d6) δ 9.06-8.97(1H, m), 7.42-7.32(2H, m), 7.23-6.93(6H, m), 6.03-5.93(1H, m), 4.73-4.38(2H, m), 3.81,3.73(3H, 2 x S), 3.80-3.40(2H, m), 3.00-2.55(4H, m), 1.92-1.69(4H, m), 1.38-1.17(4H, m).

### Example 4

### (±) Trans-N-(cyanomethyl)-2-{[4-(4-fluorobenzyl)piperazin-1-yl]carbonyl}cyclohexanecarboxamide

A mixture of (±)trans-1,2-cyclohexanedicarboxylic anhydride (0.4g), N,N-diisopropylethylamine (0.34g) and 1-(4-fluorobenzyl)piperazine (0.5g) in tetrahydrofuran (15ml) was stirred at room temperature for 18 hours. At the end of this time the reaction mixture was treated with further N,N-diisopropylethylamine (0.84g) followed by aminoacetonitrile hydrochloride (0.36g), 1-hydroxybenzotriazole (0.53g) and finally N-(3-dimethylaminopropyl)-N'-ethylcarbodiimide hydrochloride (0.75g). The mixture was stirred for 18 hours and subsequently partitioned between ethyl acetate and aqueous sodium bicarbonate, the organics dried (MgSO4) and evaporated under reduced pressure. The residue was purified by chromatography on silica, eluting with a mixture of triethylamine (0.6%), methanol (2%) and dichloromethane (97.4%). Yield 0.045g
MS: APCI(+ve) 387(M+1)
1H NMR: (DMSO-d6) δ 8.46(1H, t), 7.36-7.31(2H, m), 7.17-7.11(2H, m), 4.03(2H, d), 3.50-3.35(6H, m), 2.90-2.80(1H, m), 2.41-2.24(4H, m), 1.80-1.77(1H, m), 1.73-1.65(3H, m), 1.32-1.15(4H, m).

### Example 5

### (±) Trans-N-[cyano(2-methoxyphenyl)methyl]-2-[(4-methylpiperazin-1-yl)carbonyl]cyclohexanecarboxamide

### (i) (±) Trans-2-({[cyano(2-methoxyphenyl)methyl]amino}carbonyl) cyclohexanecarboxylic acid

A mixture of (±) trans-1,2-cyclohexanedicarboxylic anhydride (3.0g), N,N-diisopropylethylamine (5.03g) and (±) 2-(2-methoxyphenyl)aminoacetonitrile hydrochloride (3.87g) in tetrahydrofuran (50ml) was stirred at room temperature for 18 hours. The solvent was removed under reduced pressure and the residue dissolved in water. The cooled (0°C) aqueous solution was acidified by dropwise addition of dilute aqueous hydrochloric acid and the resultant mixture extracted with ethyl acetate. The organic layer was washed with aqueous brine, dried (MgSO4), and evaporated under reduced pressure. The residue was purified by tritration with ethyl acetate (2 x 30ml). Yield 0.53g
MS: APCI(+ve) 317(M+1)

### (ii) (1R,2R)-N-[Cyano(2-methoxyphenyl)methyl]-2-[(4-methylpiperazin-1-yl)carbonyl]cyclohexane carboxamide

A solution of the product from step (i) (0.38g), 1-methylpiperazine (0.18g), N,N-diisopropylethylamine (0.23g) and 1-hydroxybenzotriazole (0.24g) in 1-methyl-2-pyrrolidinone (10ml) was treated with N-(3-dimethylaminopropyl)-N'-ethylcarbodiimide hydrochloride (0.35g) and stirred for 4 hours at room temperature. The mixture was partitioned between ethyl acetate and aqueous sodium bicarbonate, the organics washed with aqueous brine (x3), dried (MgSO4) and evaporated under reduced pressure. The residue was purified by chromatography on silica, eluting with a mixture of triethylamine (0.4%), methanol (4%) and dichloromethane (95.6%) followed by tritration of the resultant product with diethyl ether. Yield 0.035g
MS: APCI(+ve) 399(M+1)
1H NMR: (DMSO-d6) δ 8.99(1H, d), 7.44-7.38(2H, m), 7.09(1H, d), 7.00(1H, t), 6.00(1H, d), 3.85(3H, s), 3.44-3.40(2H, m), 2.90-2.82(1H, m), 2.70-2.62(1H, m), 2.24-2.21(2H, m), 2.11-2.08(4H, m), 2.03-2.01(1H, m), 1.87(1H, d), 1.76-1.66(3H, m), 1.36-1.17(4H, m).

### Example 6

### (1R,2R)-N-[Cyano(2-methoxyphenyl)methyl]-2-{[4-(4-fluorophenyl)piperazin-1-yl]carbonyl}cyclohexane carboxamide

### (i) Methyl (1R,2R)-2-{[4-(4-fluorophenyl)piperazin-1-yl]carbonyl} cyclohexanecarboxylate

A solution of (1R, 2R)-cyclohexane 1,2-dicarboxylic acid mono-methyl ester (1.0g) in 1-methyl-2-pyrrolidinone (20ml) was treated with N,N-diisopropylethylamine (1.73g) followed by 1-(4-fluorophenyl)piperazine (1.45g) and 1-hydroxybenzotriazole (1.09g). The resultant mixture was then treated with with N-(3- dimethylaminopropyl)- N'-ethylcarbodiimide hydrochloride (1.55g) and stirred for 18 hours at room temperature. The mixture was partitioned between ethyl acetate and water, the organics washed with water (x3), dried (MgSO4) and evaporated under reduced pressure. The residue was purified by chromatography on silica, eluting with a mixture of ethyl acetate (45%) and isohexane (55%). Yield 1.4g
MS: APCI(+ve) 349(M+1)

### (ii) (1R,2R)-2-{[4-(4-Fluorophenyl)piperazin-1-yl]carbonyl}cyclohexanecarboxylic acid

A solution of the product from step (i) (1.1g) in methanol (40ml) was treated with a solution of sodium hydroxide (0.25g) in water (20ml) and the resultant mixture heated at 50°C for 24 hours. The solvent was removed under reduced pressure and the residue dissolved in water and washed with diethyl ether, the aqueous layer was acidified by addition of glacial acetic acid and extracted with ethyl acetate (x2). The combined ethyl acetate layers were washed with aqueous brine, dried (MgSO4) and evaporated under reduced pressure. The residue was purified by tritration with diethyl ether. Yield 0.9g
MS: APCI(+ve) 335(M+1)

### (iii) (1R,2R)-N-[Cyano(2-methoxyphenyl)methyl]-2-{[4-(4-fluorophenyl)piperazin-1-yl]carbonyl}cyclohexanecarboxamide

A solution of the product from step (iii) (0.35g) in dichloromethane (10ml) at 0°C was treated with (±) 2-(2-methoxyphenyl)aminoacetonitrile hydrochloride (0.25g) and N,N-diisopropylethylamine (0.54g) followed by O-(7-Azabenzotriazol-1-yl)-N,N,N',N'-tetramethyluronium hexafluorophosphate (0.44g). The mixture was stirred at 0°C for 1 hour and then at room temperature for 18 hours. The mixture was partitioned between ethyl acetate and water, the organics washed with water, dried (MgSO4) and evaporated under reduced pressure. The residue was purified by chromatography on silica, eluting with a mixture of ethyl acetate (67%) and isohexane (33%). Yield 0.27g
MS: APCI(+ve) 479(M+1)
1H NMR: (CDCl₃) δ 7.39-7.23(2H, m), 7.01-6.77(7H, m), 6.06-5.97(1H, m), 3.97-3.95(4H, m), 3.80-3.36(4H, m), 3.19-3.15(1H, m), 3.10-2.47(5H, m), 1.98-1.75(3H, m), 1.65-1.26(4H, m).

### Examples 7 and 8

Examples 7 and 8 were prepared according to the general method of example 6 step (iii) using the appropriate amines.

### Example 7

### (1R,2R)-N-(4-Cyanotetrahydro-2H-pyran-4-yl)-2-{[4-(4-fluorophenyl)piperazin-1-yl]carbonyl}cyclohexane carboxamide

MS: APCI(+ve) 443(M+1)
1H NMR: (CDCl₃) δ 7.00-6.94(2H, m), 6.89-6.85(2H, m), 6.32(1H, s), 3.90-3.80(3H, m), 3.78-3.59(5H, m), 3.16-3.12(1H, m), 3.09-3.00(3H, m), 2.92-2.86(1H, m), 2.70-2.64(1H, m), 2.46-2.42(1H, m), 2.22-2.19(1H, m), 1.94-1.82(6H, m), 1.69-1.63(1H, m), 1.49-1.30(3H, m).

### Example 8

### (1R,2R)-N-(4-Cyano-1-methylpiperidin-4-yl)-2-{[4-(4-fluorophenyl)piperazin-1-yl]carbonyl}cyclohexane carboxamide

MS: APCI(+ve) 456(M+1)
1H NMR: (CDCl₃) δ 7.00-6.94(2H, m), 6.90-6.85(2H, m), 6.20(1H, s), 3.90-3.85(1H, m), 3.80-3.75(1H, m), 3.70-3.60(2H, m), 3.18-3.13(1H, m), 3.09-3.04(3H, m), 2.91-2.85(1H, m), 2.73-2.61(3H, m), 2.49-2.35(3H, m), 2.29(3H, s), 2.28-2.22(1H, m), 1.90-1.81(6H, m), 1.70-1.60(1H, m), 1.50-1.32(3H, m).

### Example 9

### (1R,2R)-N-[(1S)-1-cyano-3-methoxypropyl]-2-{[4-(4-fluorophenyl)piperazin-1-yl]carbonyl}cyclohexanecarboxamide

### (i) N~2~-(tert-butoxycarbonyl)-O-methyl-L-homoserinamide

A solution of Boc-O-methyl-L-homoserine (7.75g) in dichloromethane (100ml) was treated with carbonyldiimidazole (6.46g) and the mixture stirred for 1h at room temperature. At the end of this time concentrated aqueous ammonia (20ml) was added and stirring continued for a further 40min. The reaction mixture was washed with water followed by dilute aqueous sodium hydroxide and then with aqueous brine before being dried (MgSO4) and evaporated under reduced pressure. Yield 2.9g
1H NMR: (DMSO-d6) δ 7.22(1H, s), 6.94(1H, s), 6.76(1H, d), 3.94-3.88(1H, m), 3.20(3H, s), 1.87-1.83(1H, m), 1.69-1.64(1H, m), 1.38(9H, s).

### (ii) O-Methyl-L-homoserinamide hydrochloride

A solution of the product from step (i) (2.9g) in 1,4-dioxane (30ml) was treated with a 4.0 molar solution of HCl in 1,4-dioxane (15ml) and the mixture allowed to stand for 18h at room temperature. The resultant precipitate was filtered off and washed with diethylether. Yield 1.84g
1H NMR: (DMSO-d6) δ 8.20(3H, s), 7.94(1H, s), 7.54(1H, s), 3.76(1H, s), 3.46-3.37(2H, m), 3.24(3H, s), 2.05-1.90(2H, m).

### (iii) (1R,2R)-N-[(1S)-1-Carboxamide-3-methoxypropyl]-2-{[4-(4-fluorophenyl)piperazin-1-yl]carbonyl}cyclohexanecarboxamide

A solution of the product from step (ii) (0.36g) in 1-methyl-2-pyrrolidinone (15ml) was treated with N,N-diisopropylethylamine (0.93g) followed by (1R,2R)-2-{[4-(4-fluorophenyl)piperazin-1-yl]carbonyl}cyclohexanecarboxylic acid (prepared as described in Example 6, step (ii)) (0.6g). The reaction mixture was cooled to 0°C and treated with O-(7-Azabenzotriazol-1-yl)-N,N,N',N'-tetramethyluronium hexafluorophosphate (0.75g).

The mixture was stirred at 0°C for 1h and then at room temperature for 18h. The mixture was partitioned between ethyl acetate and water, the organics washed with water (x3), dried (MgSO4) and evaporated under reduced pressure. The residue was purified by trituration with diethylether. Yield 0.40g
MS: APCI(+ve) 449(M+1)

### (iv) (1R,2R)-N-[(1S)-1-Cyano-3-methoxypropyl]-2-{[4-(4-fluorophenyl)piperazin-1-yl]carbonyl}cyclohexanecarboxamide

Oxalyl chloride (0.34g) was added dropwise to N,N-dimethylformamide (10ml) at 0°C and the mixture stirred at this temperature for 5 minutes. Pyridine (0.42g) was then added and stirring continued for a further 5 minutes. At the end of this time the mixture was treated dropwise with a solution of the product of step (iii) (0.59g) in N,N-dimethylformamide (5ml). After stirring for 2 hours at 0°C the mixture was partitioned between ethyl acetate and water, the organics washed with water, dried (MgSO4) and evaporated under reduced pressure. The residue was purified by chromatography on silica, eluting with a mixture of ethyl acetate (80%) and isohexane (20%). Yield 0.21 g
MS: APCI(+ve) 431(M+1)
1H NMR: (DMSO-d6) δ 8.57(1H, d), 7.08-7.03(2H, m), 6.99-6.94(2H, m), 4.71(1H, q), 3.70-3.58(3H, m), 3.50-3.47(1H, m), 3.37-3.30(2H, m), 3.17(3H, s), 3.08-3.00(3H, m), 2.96-2.89(2H, m), 2.57-2.49(1H, m), 1.98-1.68(6H, m), 1.38-1.20(4H, m).

### Measurement of Cathepsin S activity.

QFRET Technology (Quenched Fluorescent Resonance Energy Transfer) was used to measure the inhibition by test compounds of Cathepsin S-mediated cleavage of the synthetic peptide Z-Val-Val-Arg-AMC. Compounds were screened at five concentrations in duplicate and the pIC₅₀ values reported.

Synthetic substrate, 20µM [final]Z-Val-Val-Arg-AMC in phosphate buffer were added to a 96 well black Optiplate. The assay plates were pre-read for compound auto fluorescence on SpectraMax Gemini at 355nM excitation and 460nM emission. 250pM [final] rHuman Cathepsin S in phosphate buffer was added and incubated for 2h at room temperature on the SpectraMax Gemini, taking readings every 20min at 355nM excitation and 460nM emission.

Activity Based template (5PTB-8) used the auto fluorescent corrected data to calculate the percentage inhibition for each compound concentration using the relevent plate controls. This data was used to construct inhibition curves and pIC₅₀ estimated by non-linear regression using a 4 parameter logistic model.

## Claims

1. Use of a compound of formula (I): 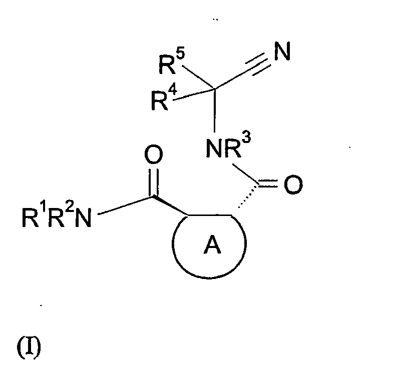 in which:
A is a 6-membered ring optionally containing a double bond and optionally containing an oxygen atom or NR group in the ring;
R is hydrogen or C₁₋₆ alkyl;
R¹ and R² are independently, C₁₋₆ alkyl or C₃₋₆ cycloalkyl both of which can optionally contain one or more O, S or NR³ groups, or R¹ and R² together with the nitrogen atom to which they are attached form a 3,4-dihydroisoquinoline ring or a 5- or 6-membered saturated ring optionally containing a further O, S or N atom and optionally substituted by a group -(CH₂)ₚ-R⁶ where p is 0 to 3 and R⁶ is C₁₋₆ alkyl, CONR⁷R⁸ where R⁷ and R⁸ are independently hydrogen, C₁₋₆ alkyl which can optionally contain one or more O, S or NR³ groups, or together with the nitrogen atom to which they are attached form a 5- or 6-membered saturated ring optionally containing a further O, S or NR³ group;
or R⁶ is a 4 to 7-membered saturated ring optionally containing one or more O, S or N atoms, or an aryl or heteroaryl group containing one to four heteroatoms selected from O, S or N, the saturated ring, aryl and heteroaryl groups all being optionally substituted by halogen, amino, hydroxy, cyano, nitro, carboxy, CONR⁷R⁸, SO₂NR⁷R⁸, SO₂R³, trifluoromethyl, NHSO₂R³, NHCOR³, C₁₋₆ alkyl, C₁₋₆ alkoxy, SR³ or NR⁹R¹⁰ where R⁹ and R¹⁰ are independently hydrogen, C₁₋₆ alkyl or together with the nitrogen atom to which they are attached form a 5- or 6-membered saturated ring optionally containing a further O, S or NR³ group;
R³ is hydrogen or C₁₋₆ alkyl;
R⁴ is hydrogen or C₁₋₆ alkyl;
R⁵ is hydrogen, C₁₋₆ alkyl or C₃₋₆ cycloalkyl both of which can optionally contain one or more O, S or NR³ groups or R⁵ is aryl or a 5- or 6-membered heteroaryl group containing one or two heteroatoms selected from O, S or N, the aryl and heteroaryl groups all being optionally substituted by halogen, amino, hydroxy, cyano, nitro, carboxy, CONR⁷R⁸, SO₂NR⁷R⁸, SO₂R³, trifluoromethyl, NHSO₂R³, NHCOR³, C₁₋₆alkyl, C₁₋₆ alkoxy, SR³ or NR⁹R¹⁰ where R⁹ and R¹⁰ are independently hydrogen, C₁₋₆ alkyl or together with the nitrogen atom to which they are attached form a 5- or 6-membered saturated ring optionally containing a further O, S or NR³ group;
or R⁴ and R⁵ together form a 5- or 6-membered saturated ring optionally containing a further O, S or NR³ group and optionally substituted by, C₁₋₆ alkyl;
and pharmaceutically acceptable salts or solvates thereof, in the manufacture of a medicament for use in the treatment of inflammation and immune disorders in a warm blooded animal, such as man.

2. Use according to claim 1 in which the disorder is asthma, rheumatoid arthritis, COPD, multiple sclerosis, Crohn's disease, Alzheimers or pain.

3. Use according to claim 1 or 2 in which A is a cyclohexane ring.

4. Use according to any one of claims 1 to 3 in which R¹ and R² together with the nitrogen atom to which they are attached form an unsubstituted morpholine ring or a piperidine ring substituted by a group -(CH₂)ₚ-R⁶ where p and R⁶ are as defined in claim 1

5. Use according to any one of claims 1 to 4 in which R³ is hydrogen.

6. Use according to any one of claims 1 to 5 in which R⁴ is hydrogen.

7. Use according to any one of claims 1 to 6 in which R⁵ is hydrogen or phenyl optionally substituted by C₁₋₆ alkyl or C₁₋₆ alkoxy.

8. Use according to any one of claims 1 to 7 where the compound of formula (I) is selected from:
(1R,2R)-N-[Cyano(2-methoxyphenyl)methyl]-2-(morpholin-4-ylcarbonyl)cyclohexanecarboxamide,
(1R,2R)-N-[Cyano(2-memoxyphenyl)methyl]-2-{[4-(4-fluorobenzyl)piperazin-1-yl]carbonyl}cyclohexane carboxamide,
(1R,2R)-N-[Cyano(2-methoxyphenyl)methyl]-2-(3,4-dihydroisoquinolin-2(1H)-ylcarbonyl)cyclohexane carboxamide,
(±) Trans-N-(cyanomethyl)-2-{[4-(4-fluorobenzyl)piperazin-1-yl]carbonyl}cyclohexanecarboxamide,
(±) Trans-N-[cyano(2-methoxyphenyl)methyl]-2-[(4-methylpiperazin-1-yl)carbonyl]cyclohexanecarboxamide,
(1R,2R)-N-[Cyano(2-methoxyphenyl)methyl]-2-{[4-(4-fluorophenyl)piperazin-1-yl]carbonyl}cyclohexane carboxamide,
(1R,2R)-N-(4-Cyano-1-methylpiperidin-4-yl)-2-{[4-(4-fluorophenyl)piperazin-1-yl]carbonyl}cyclohexane carboxamide,
and pharmaceutically acceptable salts thereof.

9. A compound of formula (I) as defined in any one of claims 1 to 8 for use in therapy.

10. A pharmaceutical composition which comprises a compound of the formula (I) as defined in any one of claims 1 to 7 or a pharmaceutically acceptable salt thereof and a pharmaceutically acceptable diluent or carrier.

## Patentansprüche

1. Verwendung einer Verbindung der Formel (I): 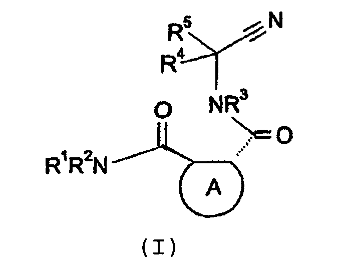 in welcher:
A für einen 6-gliedrigen Ring steht, der gegebenenfalls eine Doppelbindung enthält und gegebenenfalls im Ring ein Sauerstoffatom oder eine NR-Gruppe enthält;
R für Wasserstoff oder C₁₋₆-Alkyl steht;
R¹ und R² unabhängig voneinander für C₁₋₆-Alkyl oder C₃₋₆-Cycloalkyl stehen, die jeweils gegebenenfalls eine oder mehrere O-, S- oder NR³-Gruppen enthalten können, oder R¹ und R² zusammen mit dem Stickstoffatom, an das sie gebunden sind, einen 3,4-Dihydroisochinolinring oder einen 5- oder 6-gliedrigen gesättigten Ring, der gegebenenfalls ein weiteres O-, S- oder N-Atom enthält und gegebenenfalls durch eine Gruppe -(CH₂)ₚ-R⁶ substituiert ist, bilden, wobei p für 0 bis 3 steht und R⁶ für C₁₋₆-Alkyl oder CONR⁷R⁸ steht, wobei R⁷ und R⁸ unabhängig voneinander für Wasserstoff oder C₁₋₆-Alkyl, das gegebenenfalls eine oder mehrere O-, S- oder NR³-Gruppen enthalten kann, stehen, oder zusammen mit dem Stickstoffatom, an das sie gebunden sind, einen 5-oder 6-gliedrigen gesättigten Ring bilden, der gegebenenfalls eine weitere O-, S- oder NR³-Gruppe enthält;
oder R⁶ für einen 4- bis 7-gliedrigen gesättigten Ring, der gegebenenfalls ein oder mehrere O-, S-oder N-Atome enthält, oder eine Aryl- oder Heteroaryl-Gruppe, die 1 bis 4 Heteroatome ausgewählt aus O, S und N enthält, steht, wobei der gesättigte Ring und die Aryl- und Heteroarylgruppen jeweils gegebenenfalls durch Halogen, Amino, Hydroxy, Cyano, Nitro, Carboxy, CONR⁷R⁸, SO₂NR⁷R⁸, SO₂R³, Trifluormethyl, NHSO₂R³, NHCOR³, C₁₋₆-Alkyl, C₁₋₆-Alkoxy, SR³ oder NR⁹R¹⁰ substituiert sein können, wobei R⁹ und R¹⁰ unabhängig voneinander für Wasserstoff oder C₁₋₆₋Alkyl stehen oder zusammen mit dem Stickstoffatom, an das sie gebunden sind, einen 5- oder 6-gliedrigen gesättigten Ring bilden, der gegebenenfalls eine weitere O-, S- oder NR³-Gruppe enthält;
R³ für Wasserstoff oder C₁₋₆-Alkyl steht;
R⁴ für Wasserstoff oder C₁₋₆-Alkyl steht;
R⁵ für Wasserstoff oder C₁₋₆-Alkyl oder C₃₋₆₋Cycloalkyl steht, die beide jeweils gegebenenfalls eine oder mehrere O-, S- oder NR³-Gruppen enthalten können, oder R⁵ für Aryl oder eine 5- oder 6-gliedrige Heteroarylgruppe mit einem oder zwei Heteroatomen ausgewählt aus O, S und N steht, wobei die Aryl- und Heteroarylgruppen jeweils gegebenenfalls durch Halogen, Amino, Hydroxy, Cyano, Nitro, Carboxy, CONR⁷R⁸, SO₂NR⁷R⁸, SO₂R³, Trifluormethyl, NHSO₂R³, NHCOR³, C₁₋₆-Alkyl, C₁₋₆₋Alkoxy, SR³ oder NR⁹R¹⁰ substituiert sind, wobei R⁹ und R¹⁰ unabhängig voneinander für Wasserstoff oder C₁₋₆-Alkyl stehen oder zusammen mit dem Stickstoffatom, an das sie gebunden sind, einen 5-oder 6-gliedrigen gesättigten Ring bilden, der gegebenenfalls eine weitere O-, S- oder NR³-Gruppe enthält;
oder R⁴ und R⁵ zusammen einen 5- oder 6-gliedrigen gesättigten Ring bilden, der gegebenenfalls eine weitere O-, S- oder NR³-Gruppe enthält und gegebenenfalls durch C₁₋₆-Alkyl substituiert ist;
und pharmazeutisch annehmbaren Salzen oder Solvaten davon bei der Herstellung eines Medikaments zur Verwendung bei der Behandlung von Entzündungen und Immunstörungen in einem Warmblüter wie dem Menschen.

2. Verwendung nach Anspruch 1, wobei es sich bei der Erkrankung um Asthma, rheumatoide Arthritis, COPD, multiple Sklerose, Morbus Crohn, Alzheimer-Krankheit oder Schmerzen handelt.

3. Verwendung nach Anspruch 1 oder 2, wobei A für einen Cyclohexanring steht.

4. Verwendung nach einem der Ansprüche 1 bis 3, wobei R¹ und R² zusammen mit dem Stickstoffatom, an das sie gebunden sind, einen unsubstituierten Morpholinring oder einen durch eine -(CH₂)ₚ-R⁶⁻Gruppe substituierten Piperidinring bilden, wobei p und R⁶ wie in Anspruch 1 definiert sind.

5. Verwendung nach einem der Ansprüche 1 bis 4, wobei R³ für Wasserstoff steht.

6. Verwendung nach einem der Ansprüche 1 bis 5, wobei R⁴ für Wasserstoff steht.

7. Verwendung nach einem der Ansprüche 1 bis 6, wobei R⁵ für Wasserstoff oder gegebenenfalls durch C₁₋₆₋Alkyl oder C₁₋₆-Alkoxy substituiertes Phenyl steht.

8. Verwendung nach einem der Ansprüche 1 bis 7, wobei die Verbindung der Formel (I) ausgewählt ist aus:
(1R, 2R)-N-[Cyano(2-methoxyphenyl)methyl]-2-(morpholin-4-ylcarbonyl)cyclohexancarbonsäureamid,
(1R, 2R)-N-[Cyano(2-methoxyphenyl)methyl]-2-{[4-(4-fluorbenzyl)piperazin-1-yl]carbonyl}cyclohexancarbonsäureamid,
(1R, 2R)-N-[Cyano(2-methoxyphenyl)methyl]-2-(3,4-dihydroisochinolin-2-(1H)-ylcarbonyl)cyclohexancarbonsäureamid,
(±)-trans-N-(Cyanomethyl)-2-{[4-(4-fluorbenzyl)-piperazin-1-yl]carbonyl}cyclohexancarbonsäureamid,
(±)-trans-N-[Cyano(2-methoxyphenyl)methyl]-2-[(4-methylpiperazin-1-yl)carbonyl]cyclohexancarbonsäureamid,
(1R, 2R)-N-[Cyano(2-methoxyphenyl)methyl]-2-{[4-(4-fluorphenyl)piperazin-1-yl]carbonyl}cyclohexancarbonsäureamid,
(1R, 2R)-N-(4-Cyano-1-methylpiperidin-4-yl)-2-{[4-(4-fluorphenyl)piperazin-1-yl]carbonyl}cyclohexancarbonsäureamid,
und deren pharmazeutisch annehmbaren Salzen.

9. Verbindung der Formel (I) nach einem der Ansprüche 1 bis 8 zur Verwendung in der Therapie.

10. Pharmazeutische Zusammensetzung, enthaltend eine Verbindung der Formel (I) nach einem der Ansprüche 1 bis 7 oder ein pharmazeutisch annehmbares Salz davon und ein pharmazeutisch annehmbares Verdünnungsmittel oder einen pharmazeutisch annehmbaren Träger.

## Revendications

1. Utilisation d'un composé de formule (I) : 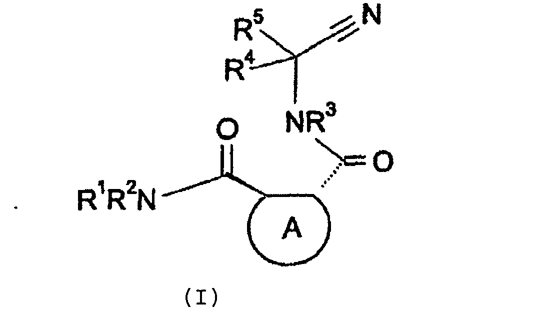 dans laquelle :
A est un cycle à 6 chaînons contenant éventuellement une double liaison et contenant éventuellement un atome d'oxygène ou un groupement NR dans le cycle ;
R est hydrogène ou C₁₋₆ alkyle ;
R¹ et R² sont indépendamment C₁₋₆ alkyle ou C₃₋₆ cycloalkyle qui peuvent tous les deux éventuellement contenir un ou plusieurs groupements O, S ou NR³, ou R¹ et R² ensemble avec l'atome d'azote auquel ils sont liés forment un cycle 3,4-dihydroisoquinoléine ou un cycle saturé à 5 ou 6 chaînons contenant éventuellement un autre atoine, O, S ou N et éventuellement substitué par un groupement - (CH2) p-R6 où p est de 0 à 3 et R⁶ est C₁₋₆ alkyle, CONR⁷R⁸ où R⁷ et R⁸ sont indépendamment hydrogène, C₁₋₆ alkyle qui peut contenir éventuellement un ou plusieurs groupements O, S ou NR³, ou ensemble avec l'atome d'azote auquel ils sont liés forment un cycle saturé à 5 ou 6 chaînons contenant éventuellement un autre groupement O, S ou NR³ ; ou R⁶ est un cycle saturé à 4 à 7 chaînons contenant éventuellement un ou plusieurs atomes O, S ou N, ou un groupement aryle ou hétéroaryle contenant de 1 à 4 hétéroatomes choisis parmi O, S ou N, le cycle saturé et les groupements aryle et hétéroaryle étant tous éventuellement substitués par halogène, amino, hydroxy, cyano, nitro, carboxy, CONR⁷R⁸, SO₂NR⁷R⁸, SO₂R³, trifluorométhyle, NHSO₂R³, NHCOR³, C₁₋₆ alkyle, C₁₋₆ alcoxy, SR³ ou NR⁹R¹⁰ où R⁹ et R¹⁰ sont indépendamment hydrogène, C₁₋₆ alkyle ou ensemble avec l'atome d'azote auquel ils sont liés forment un cycle saturé à 5 ou 6 chaînons contenant éventuellement un autre groupement O, S ou NR³ ;
R³ est hydrogène ou C₁₋₆ alkyle ;
R⁴ est hydrogène ou C₁₋₆ alkyle ;
R⁵ est hydrogène, C₁₋₆ alkyle ou C₃₋₆ cycloalkyle qui peuvent tous les deux éventuellement contenir un ou plusieurs groupements O, S ou NR³ ou R⁵ est aryle ou un groupement hétéroaryle à 5 ou 6 chaînons contenant un ou deux hétéroatomes choisis parmi O, S ou N, les groupements aryle et hétéroaryle étant tous éventuellement substitués par halogène, amino, hydroxy, cyano, nitro, carboxy, CONR⁷R⁸, SO₂NR⁷R⁸, SO₂R³, trifluorométhyle, NHSO₂R³, NHCOR³, C₁₋₆ alkyle, C₁₋₆ alcoxy, SR³ ou NR⁹R¹⁰ où R⁹ et R¹⁰ sont indépendamment hydrogène, C₁₋₆ alkyle ou ensemble avec l'atome d'azote auquel ils sont liés forment un cycle saturé à 5 ou 6 chaînons contenant éventuellement un autre groupement O, S ou NR³ ;
ou R⁴ et R⁵ ensemble forment un cycle saturé à 5 ou 6 chaînons contenant éventuellement un autre groupement O, S ou NR³ et éventuellement substitué par C₁₋₆ alkyle ;
et les sels ou solvates pharmaceutiquement acceptables de celui-ci, dans la fabrication d'un médicament destiné à être utilisé dans le traitement des troubles inflammatoires et immunitaires chez un animal à sang chaud, tel que l'homme.

2. Utilisation selon la revendication 1, **caractérisée en ce que** le trouble est l'asthme, la polyarthrite rhumatoïde, la BPCO, la sclérose en plaques, la maladie de Crohn, la maladie d'Alzheimer ou la douleur.

3. Utilisation selon la revendication 1 ou 2, **caractérisée en ce que** A est un cycle cyclohexane.

4. Utilisation selon l'une quelconque des revendications 1 à 3, **caractérisée en ce que** R¹ et R² ensemble avec l'atome d'azote auquel ils sont liés forment un cycle morpholine non substitué ou un cycle pipéridine substitué par un groupement -(CH₂)_{P}-R⁶ où p et R⁶ sont tels que définis dans la revendication 1.

5. Utilisation selon l'une quelconque des revendications 1 à 4, **caractérisée en ce que** R³ est hydrogène.

6. Utilisation selon l'une quelconque des revendications 1 à 5, **caractérisée en ce que** R⁴ est hydrogène.

7. Utilisation selon l'une quelconque des revendications 1 à 6, **caractérisée en ce que** R⁵ est hydrogène ou phényle éventuellement substitué par C₁₋₆ alkyle ou C₁₋₆ alcoxy.

8. Utilisation selon l'une quelconque des revendications 1 à 7, **caractérisée en ce que** le composé de formule (I) est choisi parmi :
le (1R, 2R)-N-[cyano(2-méthoxyphényl)méthyl]-2-(morpholin-4-ylcarbonyl)cyclohexanecarboxamide,
le (1R, 2R)-N-[cyano(2-méthoxyphényl)méthyl]-2-{[4-(4-fluorobenzyl)pipérazin-1-yl]carbonyl}cyclohexanecarboxamide,
le (1R, 2R)-N-[cyano(2-méthoxyphényl)méthyl]-2-(3,4-dihydroisoquinoléin-2-(1H)-ylcarbonyl)-cyclohexanecarboxamide,
le (±)trans-N-(cyanométhyl)-2-([4-(4-fluorobenzyl)pipérazin-1-yl]carbonyl}cyclohexanecarboxamide,
le (±)trans-N-[cyano(2-méthoxyphényl)méthyl]-2-[(4-méthylpipérazin-1-yl)carbonyl]cyclohexanecarboxamide,
le (1R, 2R)-N-[cyano(2-méthoxyphényl)méthyl]-2-{[4-(4-fluorophényl)pipérazin-1-yl]carbonyl}cyclohexanecarboxamide,
le (1R, 2R)-N-(4-cyano-1-méthylpipéridin-4-yl)-2-{[4-(4-fluorophényl)pipérazin-1-yl]carbonyl}cyclohexanecarboxamide,
et les sels pharmaceutiquement acceptables de ceux-ci.

9. Composé de formule (I) tel que défini dans l'une quelconque des revendications 1 à 8, destiné à être utilisé en thérapie.

10. Composition pharmaceutique, **caractérisée en ce qu'**elle comprend un composé de formule (I) tel que défini dans l'une quelconque des revendications 1 à 7 ou un sel pharmaceutiquement acceptable de celui-ci et un diluant ou un support pharmaceutiquement acceptable.
